# EUROPÄISCHE PATENTSCHRIFT

(11) **EP 1 440 055 B1**
(45) Veröffentlichungstag und Bekanntmachung des Hinweises auf die Patenterteilung: **03.09.2008**
(21) Anmeldenummer: 02785231.8
(22) Anmeldetag: 17.10.2002
(51) Int. Cl.: C07C 263/10, C07C 265/08, C07C 265/10, C07C 265/12, C07C 265/04

(54) **VERFAHREN ZUR HERSTELLUNG VON ISOCYANATEN**
METHOD FOR PRODUCTION OF ISOCYANATES
PROCEDE DE PRODUCTION D'ISOCYANATES

(30) Priorität: 23.10.2001 DE 10152118
(43) Veröffentlichungstag der Anmeldung: 28.07.2004
(73) Patentinhaber: BASF SE, 67056 Ludwigshafen (DE)
(72) Erfinder: ROHDE, Thorsten, 68167 Mannheim (DE); STAMM, Armin, 55268 Nieder-Olm (DE); HENKELMANN, Jochem, 68165 Mannheim (DE)
(86) Internationale Anmeldenummer: PCT/EP2002/011624
(87) Internationale Veröffentlichungsnummer: WO 2003/042170

(56) Entgegenhaltungen:
- DE-A- 19 942 299
- US-A- 3 484 466
- US-A- 4 888 125

## Beschreibung

Die vorliegende Erfindung betrifft ein Verfahren zur Herstellung von Isocyanaten durch Umsetzung der entsprechenden primären Amine mit Phosgen in einem inerten Lösungsmittel.

Isocyanate sind großtechnische Produkte mit einer Vielzahl von Anwendungen auf dem Gebiet der Polyurethan-Kunststoffe. Bestimmte Isocyanate finden aber auch Anwendung bei der Herstellung pharmazeutischer Wirkstoffe.

Die Synthese von Isocyanaten durch Umsetzung von Aminen mit Phosgen ist seit langen bekannt. Prinzipiell werden zwei Verfahren in der Literatur beschrieben, von denen das eine bei Normaldruck und das andere bei erhöhtem Druck durchgeführt wird. Die Phosgenierung unter erhöhtem Druck ist nachteilig, da sie zur Beherrschung des erhöhten Sicherheitsrisikos, der Freisetzung von Phosgen, einen stark erhöhten apparativ technischen Aufwand erfordert.

Für Sulfonylisocyanate ist aus US 3,371,114 und US 3,484,466 ein Herstellungsverfahren bei Normaldruck bekannt, bei dem eine Lösung eines Sulfonylamids und eines Isocyanats als Katalysator in einem inerten Lösungsmittel mit Phosgen umgesetzt wird. Dabei wird intermediär der entsprechende Sulfonylharnstoff gebildet, welcher mit Phosgen zum gewünschten Sulfonylisocyanat reagiert.

Alkyl- und Arylisocyanate werden üblicherweise nach dem beispielsweise in Ullmann's Encyclopedia of Industrial Chemistry, 6th edition, 2000 electronic release, Chapter "ISOCYANATES, ORGANIC - Production" beschriebenen Phosgenierungsverfahren in zwei Phasen bei Normaldruck aus den entsprechenden Aminen hergestellt. In der ersten Phase, der Kaltphosgenierung, wird das Amin mit einem Phosgenüberschuß in stark verdünnter Lösung und bei tiefen Temperaturen zum entsprechenden Carbaminsäurechlorid umgesetzt, aus dem in der zweiten Phase bei erhöhter Temperatur, der Heißphosgenierung, das Isocyanat entsteht. Aliphatische und cycloaliphatische primäre Amine sind dabei aufgrund ihrer gegenüber aromatischen Aminen erhöhten Basizität schlechter zu phosgenieren und führen zu einem vermehrten Anfall an Nebenprodukten. Nachteilig an diesen Verfahren ist neben der Phosgenierung in zwei Phasen, vor allem die Bildung einer intermediären Feststoffsuspension aus schwerlöslichem Carbaminsäurechlorid und Aminhydrochlorid, die wiederum eine erhöhte Verdünnung des Reaktionsmediums erforderlich macht, um Ablagerungen und Verstopfungen von Anlagenteilen zu verhindern. Aufgrund des vorhandenen Feststoffanfalls kann dieses Verfahren bei Normaldruck nicht kontinuierlich durchgeführt werden. Weiterhin entsteht ein symmetrisch N,N'-disubstituierter Harnstoff als Nebenprodukt, dessen Bildung nur um den Preis drastisch verringerter Raum/Zeit-Ausbeuten unterdrückt werden kann.

Aliphatische und cycloaliphatische Amine werden häufig in Form ihrer Salze in die Kalt/Heißphosgenierung eingesetzt. Diese Salze sind jedoch im Reaktionsmedium schwer löslich, so daß zusätzliche Reaktionsstufen und sehr lange Reaktionszeiten erforderlich werden.

Weiterhin lehren US 3,440,268 und US 3,492,331 die Umsetzung primärer Amine mit Phosgen in Gegenwart von N,N-disubstituiertem Formamid, N-Alkyllactam, N,N-disubstituiertem N'-Aryl-formamidin und N,N,N',N'-tetrasubstituiertem N"-Aryl-guanidin als Katalysator. Aus H. Ulrich, Chemistry & Technology of Isocyanates, Wiley & Sons, 1996, Seiten 328 bis 330 ist die Umsetzung primärer Amine mit Phosgen in Gegenwart von tertiären Aminen, Tetramethylharnstoff und Carbonyldiimidazol als Katalysator sowie aus CS 262 295 in Gegenwart von N,N'-Diazabicyclo[2,2,2]oktan als Katalysator bekannt. Die genannten Verbindungen müssen zum Teil in äquimolaren Mengen eingesetzt werden und bilden unter den Reaktionsbedingungen schwer lösliche Salze in Form der Katalysator-Hydrochlorid-Addukte. Des weiteren bildet sich aus dem eingesetzten Amin und dem gebildeten Chlorwasserstoff schwerlösliches Amin-Hydrochlorid.

WO 01/17951 lehrt die Herstellung von Isocyanaten durch Phosgenierung der entsprechenden primären Amine in Gegenwart eines Monoisocyanats, welches vor Beginn der Umsetzung in einem inerten Lösungsmittel vorgelegt und mit Phosgen versetzt wird. Nachteilig an diesem Verfahren ist, dass die bevorzugt einzusetzenden niedermolekularen aliphatischen Isocyanate toxisch sind und hohe Sicherheitsvorkehrungen erfordern.

Es bestand daher die Aufgabe, ein Verfahren zur Herstellung von Isocyanaten zu finden, welches die genannten Nachteile nicht mehr besitzt, sowohl kontinuierlich als auch diskontinuierlich durchführbar ist, keine oder nur eine unwesentliche Bildung schwerlöslicher Komponenten zeigt, ohne stark toxischem Katalysator durchführbar ist, die Umsetzung in nur einer Reaktionsstufe ermöglicht und auch unter milden Temperaturen und Drücken zu einem hohen Umsatz, einer hohen Selektivität und einer hohen Raum/Zeit-Ausbeute führt.

Demgemäß wurde ein Verfahren zur Herstellung von Isocyanaten durch Umsetzung der entsprechenden primären Amine mit Phosgen in einem inerten Lösungsmittel bei einer Temperatur von 20 bis 200°C und einem Druck von 0,05 bis 0,5 MPa abs gefunden, das dadurch gekennzeichnet ist, dass man 0,01 bis 50 Mol-% eines Sulfonsäureamids, bezogen auf die Gesamtmenge an primärem Amin und gebildetem Isocyanat in der Reaktionslösung, einsetzt.

Das beim erfindungsgemäßen Verfahren einsetzbare Sulfonsäureamid besitzt die allgemeine Formel (I)

R¹-SO₂-NH₂ (I),

in der der Rest R¹ für einen Kohlenstoff enthaltenden organischen Rest steht.

Unter einem Kohlenstoff enthaltenden organischen Rest ist ein unsubstituierter oder substituierter, aliphatischer, aromatischer oder araliphatischer Rest zu verstehen. Dieser kann ein oder mehrere Heteroatome, wie etwa Sauerstoff, Stickstoff, Schwefel oder Phosphor enthalten, beispielsweise -O-, -S-, -NR-, -CO-, -N=, -SiR₂-, -PR- und/oder -PR₂ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Bei dem Kohlenstoff enthaltenden organischen Rest kann es sich um einen einwertigen oder auch um einen zwei- oder dreiwertigen Rest handeln.

Bevorzugt steht der Rest R¹ für
- einen unverzweigten oder verzweigten, acyclischen oder cyclischen, unsubstituierten oder substituierten Alkylrest mit 1 bis 30 aliphatischen Kohlenstoffatomen, bei dem eine oder mehrere der CH₂-Gruppen auch durch Heteroatome, wie -O- oder -S-, oder durch Heteroatom enthaltende Gruppen, wie -CO-, -NR- oder -SiR₂-, ersetzt sein können und bei dem ein oder mehrere der Wasserstoffatome durch Substituenten, wie beispielsweise Arylgruppen oder funktionelle Gruppen ersetzt sein können; oder für
- einen unsubstituierten oder substituierten aromatischen Rest mit 3 bis 30 Kohlenstoffatomen und einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl-, Arylgruppen oder funktionelle Gruppen ersetzt sein können.

Besonders bevorzugt werden Sulfonsäureamide (I) eingesetzt, deren Rest R¹
- einen unverzweigten oder verzweigten C₁- bis C₂₀-Alkylrest, wie beispielsweise Methyl, Ethyl, 1-Propyl, 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl, 3-Methyl-3-pentyl oder 2-Methoxy-2-propyl;
- einen unverzweigten oder verzweigten C₅- bis C₂₀-Cycloalkylrest, wie beispielsweise Cyclopentyl, Cyclohexyl oder Cyclooctyl; oder
- einen unsubstituierten oder mit einem oder mehreren C₁- bis C₄-Alkylresten substituierten C₆- bis C₂₀-Aryl- oder C₃- bis C₂₀-Heteroaryl-Rest, wie beispielsweise Phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl, 2-Methoxyphenyl, 3-Methoxyphenyl, 4-Methoxyphenyl, 2-Pyridyl, 3-Pyridyl, 4-Pyridyl, 3-Pyridazinyl, 4-Pyridazinyl, 5-Pyridazinyl, 2-Pyrimidinyl, 4-Pyrimidinyl, 5-Pyrimidinyl, 2-Pyrazinyl, 2-(1,3,5-Triazin)yl, 1-Naphthyl, 2-Naphthyl, 2-Chinolyl, 8-Chinolyl, 1-Isochinolyl oder 8-Isochinolyl;
bedeutet.

Ganz besonders bevorzugt werden Sulfonsäureamide (I) eingesetzt, deren Rest R¹
- einen unverzweigten oder verzweigten C₁- bis C₁₀-Alkylrest;
- einen unverzweigten oder verzweigten C₅- bis C₁₀-Cycloalkylrest; oder
- einen unsubstituierten oder mit einem oder mehreren C₁- bis C₄-Alkylresten substituierten C₆- bis C₁₂-Aryl-Rest;
bedeutet.

Insbesondere setzt man beim erfindungsgemäßen Verfahren ein aromatisches Sulfonsäureamid, ganz besonders bevorzugt p-Toluolsulfonsäureamid ein.

Das Sulfonsäureamid wird in einer katalytischen Menge von 0,01 bis 50 Mol-%, bezogen auf die Gesamtmenge an primärem Amin und gebildetem Isocyanat in der Reaktionslösung, eingesetzt. Bei der Berechnung der Gesamtmenge an primärem Amin und gebildetem Isocyanat sind die Molmengen des noch nicht-umgesetzten Edukts (primäres Amin) und des bereits gebildeten Produkts (Isocyanat) sowie gegebenenfalls vorhandener Zwischenprodukte zu addieren. Beim erfindungsgemäßen Verfahren setzt man bevorzugt 0,01 bis 25 Mol-%, besonders bevorzugt 0,5 bis 20 Mol-% und ganz besonders bevorzugt 1 bis 15 Mol-% Sulfonsäureamid, bezogen auf die Gesamtmenge an primärem Amin und gebildetem Isocyanat in der Reaktionslösung, ein.

Beim erfindungsgemäßen Verfahren legt man im Allgemeinen das Sulfonsäureamid in einem inerten Lösungsmittel vor. Unter einem inerten Lösungsmittel sind Lösungsmittel zu verstehen, welche gegenüber dem eingesetzten primären Amin, dem Phosgen, dem gebildeten Isocyanat und dem verwendeten Sulfonsäureamid chemisch inert sind. "Chemisch inert" heißt, daß sich die Verdünnungsmittel unter den gewählten Bedingungen nicht mit den genannten Stoffen chemisch umsetzen. Bevorzugt setzt man aromatische oder aliphatische Kohlenwasserstoffe und besonders bevorzugt einfach oder mehrfach substituierte aromatische Kohlenwasserstoffe, wie beispielsweise Toluol, O-, m-, p-Xylol, Ethylbenzol, Chlorbenzol oder O-, m-, p-Dichlorbenzol ein. Ganz besonders bevorzugt sind O-, m- oder p-Xylol, Chlorbenzol, O-, m-, p-Dichlorbenzol oder deren Gemische.

Anschließend beginnt man im Allgemeinen mit der Einleitung von Phosgen. Es kann in flüssiger oder gasförmiger Form eingeleitet werden. In der Regel leitet man zunächst etwa 10 bis 50% der, bezogen auf das Reaktionsvolumen, theoretisch benötigten Menge an Phosgen ein.

In die mit Phosgen beladene Ausgangslösung von Sulfonsäureamid in inertem Lösungsmittel beginnt man nun, das primäre Amin zuzuführen. Phosgen wird dabei entsprechend dem Reaktionsfortgang und der Menge an zugeführten Amin weiter eingeleitet.

Die beim erfindungsgemäßen Verfahren einsetzbaren primären Amin besitzen die allgemeine Formel (II)

R²-NH₂ (II),

in der der Rest R² für einen Kohlenstoff enthaltenden organischen Rest, wie zuvor definiert, steht.

Bevorzugt steht der Rest R² für
- einen unverzweigten oder verzweigten, acyclischen oder cyclischen, unsubstituierten oder substituierten Alkylrest mit 1 bis 30 aliphatischen Kohlenstoffatomen, bei dem eine oder mehrere der_CH₂-Gruppen auch durch Heteroatome, wie -O- oder -S-, oder durch Heteroatom enthaltende Gruppen, wie -CO-, -NR- oder -SiR₂-, ersetzt sein können und bei dem ein oder mehrere der Wasserstoffatome durch Substituenten, wie beispielsweise Arylgruppen oder funktionelle Gruppen, ausgenommen -OH, -SH, und -COOH Gruppen, ersetzt sein können; oder für
- einen unsubstituierten oder substituierten aromatischen Rest mit 3 bis 30 Kohlenstoffatomen und einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl-, Arylgruppen oder funktionelle Gruppen, ausgenommen -OH, -SH und -COOH Gruppen, ersetzt sein können.

Insbesondere kann der Rest R² eine oder mehrere weitere NH₂-Gruppen tragen, so dass als primäre Amine explizit auch Oligoamine mit zwei oder mehreren NH₂-Gruppen umfasst sind. Besonders bevorzugt setzt man als primäres Amin ein Monoamin mit einer NH₂-Gruppe oder ein Diamin mit zwei NH₂-Gruppen mit jeweils 1 bis 20 Kohlenstoffatomen ein.

Als Beispiele geeigneter acyclischer und gegebenenfalls substituierter Alkylreste Reste für R² seien Methyl, Ethyl, 1-Propyl, - 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl, 3-Methyl-3-pentyl, 1-Octyl, 1-Decyl, 2-Aminoethyl, 3-Aminopropyl, 4-Aminobutyl, 5-Aminopentyl, 6-Aminohexyl, 8-Aminooctyl, Phenylmethyl, 1-Phenylethyl, 1-Phenylpropyl oder 1-Phenylbutyl genannt.

Als Beispiele cyclischer und gegebenenfalls substituierter Cycloalkylreste Reste für R² seien Cyclopentyl, Cyclohexyl, Cyclooctyl oder 3-Aminomethyl-3,5,5-trimetyhlcyclohexyl genannt.

Als Beispiele aromatischer und heteroaromatischer und gegebenenfalls substituierter Reste für R² seien Phenyl, O-, m-, p-Tolyl und Aminotolyle genannt.

Es sei betont, dass als primäre Amine auch enantiomerenreine, optisch aktive Verbindungen oder Gemische davon mit einem an der NH₂-Gruppe gebundenen optisch aktiven Kohlenstoffatom eingesetzt werden können. Das erfindungsgemäße Verfahren führt während der Reaktion und der Aufarbeitung vorteilhafterweise einem lediglich geringen Anteil an Racemisierung, der im Allgemeinen unter 2% liegt.

Ganz besonders bevorzugt setzt man bei erfindungsgemäßen Verfahren als primäres Amin 1,6-Diaminohexan, Cyclohexylamin, Isophorondiamin (3-Aminomethyl-3,5,5-trimetyhlcyclohexylamin), Anilin, Diaminotoluole, Diphenylmethan-4,4'-diamin, R-(+)- und S(-)-Phenylethylamin ein.

Das Molverhältnis zwischen der Gesamtmenge an zugeführtem Phosgen und den zu phosgenierenden primären Amin-Gruppen beträgt im Allgemeinen 1 bis 10, bevorzugt 1 bis 5 und besonders bevorzugt 1 bis 2, insbesondere 1,2 bis 1,8.

Die Menge an inertem Lösungsmittel beträgt im Allgemeinen 100 bis 2000 Gew.-% und bevorzugt 300 bis 1000 Gew.-%, bezogen auf die Gesamtmenge des vorliegenden primären Amins und des gebildeten Isocyanats.

Das erfindungsgemäße Verfahren führt man bei einer Temperatur von 20 bis 200°C, bevorzugt von 20 bis 150°C und besonders bevorzugt von 50 bis 100°C durch. Bei Durchführung des erfindungsgemäßen Verfahrens beträgt der Druck 0,05 bis 0,5 MPa abs und bevorzugt 0,08 bis 0,12 MPa abs.

Beim erfindungsgemäßen Verfahren legt man bevorzugt das Sulfonsäureamid in einem inerten Lösungsmittel vor, leitet darin Phosgen ein und führt in die mit Phosgen beladene Ausgangslösung das primäre Amin zu.

Nachdem die gewünschte Menge an Phosgen und Amin zugegeben wurde, wird die erhaltene Reaktionslösung in der Regel noch für eine gewisse Zeit, im Allgemeinen 30 Minuten bis 6 Stunden unter den Reaktionsbedingungen zur Nachreaktion belassen. Um das überschüssige Phosgen und dessen Reaktionsprodukte Kohlendioxid oder Chlorwasserstoff aus der Reaktionslösung zu entfernen beziehungsweise abzureichern, wird anschließend in der Regel unter intensiver Durchmischung Inertgas durchgeleitet ("Strippen").

Das erfindungsgemäße Verfahren kann prinzipiell kontinuierlich als auch diskontinuierlich durchgeführt werden, wobei eine kontinuierliche Verfahrensführung bevorzugt ist. Das Verfahren kann in einer beliebigen, für eine Umsetzung mit Phosgen geeigneten Anlage erfolgen. Als geeignete Reaktoren seien beispielsweise Rührkessel genannt.

Der Reaktionsaustrag wird im Allgemeinen mit den bekannten Methoden aufgearbeitet. Vorzugsweise wird das gewünschte Isocyanat durch fraktionierte Destillation isoliert. Das als Katalysator eingesetzte Sulfonsäureamid wird dabei bevorzugt durch Destillation zurückgewonnen und bei einem kontinuierlichen Verfahren rückgeführt.

In einer allgemeinen Ausführungsform zur diskontinuierlichen Herstellung von Isocyanaten legt man das Sulfonsäureamid in einem inerten Lösungsmittel im Reaktor, beispielsweise einem Rührkessel, unter Rühren vor und beläd die Lösung mit Phosgen. Das Reaktionssystem wird nun auf die gewünschte Temperatur gebracht und mit der Zufuhr des Amins begonnen. Phosgen wird dabei entsprechend dem Reaktionsfortgang und der Menge an zugeführten Amin weiter eingeleitet. Nachdem die gewünschte Menge an Phosgen zugeführt wurde, wird die Reaktionslösung noch einige Zeit bei der eingestellten Temperatur unter fortgesetztem Rühren zur Nachreaktion belassen. Bei der Nachreaktion setzt sich noch in der Reaktionslösung befindliches Phosgen mit restlichem Amin um. Um das überschüssige Phosgen und dessen Reaktionsprodukte Kohlendioxid oder Chlorwasserstoff aus der Reaktionslösung zu entfernen beziehungsweise abzureichern, ist es möglich, unter intensiver Durchmischung Inertgas durchzuleiten ("Strippen"). Die erhaltene Reaktionslösung wird nun der Aufarbeitung zugeführt. Im Allgemeinen erfolgt die Aufarbeitung destillativ, gegebenenfalls unter Vakuum. Bei höhersiedenden Isocyanaten sind auch andere Reinigungsverfahren, wie beispielsweise Kristallisation möglich.

In einer allgemeinen Ausführungsform zur kontinuierlichen Herstellung von Isocyanaten legt man das Sulfonsäureamid in einem inerten Lösungsmittel im Reaktor, beispielsweise einem Rührkessel, unter Rühren vor und beläd die Lösung mit Phosgen. Das Reaktionssystem wird nun auf die gewünschte Temperatur gebracht und mit der kontinuierlichen Zufuhr des Amins begonnen. Phosgen wird dabei entsprechend dem Reaktionsfortgang und der Menge an zugeführten Amin kontinuierlich eingeleitet. Nachdem sich der Reaktorinhalt weitgehend zum Isocyanat umgesetzt hat, gleicht man die Mengen an Amin und Phosgen derart an, daß beide im Wesentlichen in stöchiometrisch erforderlichem Verhältnis zugeführt werden. Eine der zugefahrenen Menge entsprechende Menge des Reaktionsvolumens wird dem Reaktionsapparat, beispielsweise über eine Standhaltung oder einem Überlauf, entnommen. Die entnommene Reaktionslösung wird in einem nachgeschalteten Behälter, beispielsweise einem Rührkessel, zur Nachreaktion aufgefangen. Nachdem auch der nachgeschaltete Behälter durch den Reaktionsaustrag gefüllt wurde, wird der Überlauf gegebenenfalls von den Koppelprodukten Kohlendioxid und Chlorwasserstoff wie oben beschrieben durch Strippen befreit und der Aufarbeitung zugeführt. Die Aufarbeitung kann beispielsweise destillativ durchgeführt werden.

Das erfindungsgemäße Verfahren ermöglicht die kontinuierliche oder diskontinuierliche Herstellung von Isocyanaten durch Phosgenierung von primären Aminen. Es ermöglicht gegenüber den bekannten, katalysatorfreien Verfahren die Durchführung der eigentlichen Umsetzung in nur einer einzigen Reaktionsstufe unter milden Temperaturen und Drücken, wobei ein hoher Umsatz an primärem Amin, eine hohe Selektivität und eine hohe Raum/Zeit-Ausbeute an Isocyanat erreicht wird. Gegenüber den bekannten, katalysatorfreien Verfahren und den bekannten Verfahren in Gegenwart eines Katalysators weist das erfindungsgemäße Verfahren keine oder nur eine unwesentliche Tendenz zur Bildung schwerlöslicher Komponenten auf. Das Risiko zur Ablagerung an und Verstopfung von Anlagenteilen wird dadurch deutlich minimiert, was beim Umgang mit dem toxischen Phosgen einen entscheidenden Sicherheitsgewinn darstellt. Ferner wird duch die ausbleibende oder nur unwesentliche Bildung schwerlöslicher Komponenten die Durchführung eines kontinuierlichen Verfahrens erst ermöglicht und signifikante Vorteile bei der anschließenden Aufarbeitung erzielt.

Die beim erfindungsgemäßen Verfahren einzusetzenden Sulfonsäureamide sind in der Regel nicht oder nur minder toxisch, was vor allem einen Vorteil gegenüber dem in WO 01/17951 beschriebenen Einsatz eines aliphatischen Monoisocyanats darstellt.

### Beispiele

### Versuchsanordnung

Die Versuchsanordnung umfasste ein 1 L Glasgefäß mit einem Rührer, einer Thermostatierung, einem Einleitungsrohr für das gasförmige Phosgen und einer zweiteiligen Kühlerkaskade. Die zweiteilige Kühlerkaskade umfasst einen Intensivkühler, welcher auf -10°C temperiert wurde und einen Kohlensäurekühler, welcher auf -78°C temperiert wurde. Die Versuche wurden bei Atmosphärendruck durchgeführt.

### Vergleichsbeispiel 1

500 g Chlorbenzol wurden im Glasgefäß vorgelegt und bei Raumtemperatur 40 g Phosgen eingegast. Anschließend wurde der Reaktionsansatz auf 77°C erhitzt, wobei sich ein kräftiger Phosgenrückfluß einstellte. Unter intensivem Rühren nun wurden bei 77 bis 80°C innerhalb von 3 Stunden insgesamt 99,2 g Cyclohexylamin (1 Mol), gelöst in 200 g Chlorbenzol, und gleichzeitig weitere 92 g Phosgen zugeführt. Nach beendeter Zufuhr wurde das System zur Nachreaktion eine weitere Stunde ohne Phosgenzufuhr bei 77 bis 80°C belassen und anschließend das restliche, nicht-umgesetzte Phosgen mit Stickstoff bei 50°C herausgestrippt. Bei dem erhaltenen Reaktionsgemisch handelte es sich um eine Suspension, von der der enthaltene Feststoff durch Filtration abgetrennt wurde. Es konnten dabei 15 g Feststoff, bei dem es sich laut IR-spektroskopischer Analyse hauptsächlich um Aminhydrochlorid handelte, isoliert werden. Der filtrierte Rohaustrag wurde destillativ aufgearbeitet. Nach Abtrennung des Lösungsmittels und fraktionierter Destillation wurden 88,8 g Cyclohexylisocyanat (0,710 Mol) isoliert. Dies entspricht 71,0% der theoretischen Ausbeute.

### Beispiel 2 (erfindungsgemäß)

500 g Chlorbenzol und 4,3 g p-Toluolsulfonsäureamid (0,025 Mol) wurden im Glasgefäß vorgelegt und bei Raumtemperatur 33 g Phosgen eingegast. Anschließend wurde der Reaktionsansatz auf 79°C erhitzt, wobei sich ein kräftiger Phosgenrückfluß einstellte. Unter intensivem Rühren nun wurden bei 78 bis 81°C innerhalb von 3 Stunden insgesamt 99,2 g Cyclohexylamin (1 Mol), gelöst in 200 g Chlorbenzol, und gleichzeitig weitere 122 g Phosgen zugeführt. Nach beendeter Zufuhr wurde das System zur Nachreaktion eine weitere Stunde ohne Phosgenzufuhr bei 78 bis 81°C belassen und anschließend das restliche, nicht-umgesetzte Phosgen mit Stickstoff bei 50°C herausgestrippt. Bei dem erhaltenen Reaktionsgemisch handelte es sich um eine dünnflüssige Suspension, von der der enthaltene Feststoff durch Filtration abgetrennt wurde. Es konnten dabei < 0,5 g Feststoff isoliert werden. Cyclohexylisocyanat und wurde destillativ aufgearbeitet. Nach Abtrennung des Lösungsmittels und fraktionierter Destillation wurden 105,1 g Cyclohexylisocyanat (0,833 Mol) isoliert. Dies entspricht 83,3% der theoretischen Ausbeute.

Das erfindungsgemäße Beispiel 2 unter Einsatz von p-Toluolsulfonsäureamid zeigt gegenüber dem Vergleichsbeispiel 1 ohne Einsatz eines Katalysators nur einen sehr geringen Feststoffanteil. Bei dem erfindungsgemäßen Verfahren ist somit nur sehr wenig Feststoff abzutrennen.

Durch den signifikant niedrigeren Feststoffanteil läßt sich das erfindungsgemäße Verfahren wesentlich einfacher, sicherer und problemloser durchführen. Insbesondere wird das Risiko zur Ablagerung an und Verstopfung von Anlagenteilen deutlich minimiert, was beim Umgang mit dem toxischen Phosgen einen entscheidenden Sicherheitsgewinn darstellt und die Durchführung eines kontinuierlichen Verfahrens ermöglicht.

Des weiteren zeigt das erfindungsgemäße Beispiel 2 unter Einsatzvon p-Toluolsulfonsäureamid mit 83,3% % der theoretischen Ausbeute einen wesentlich höheren Umsatz als das Vergleichsbeispiel 1 ohne Einsatz eines Katalysators mit 71,0% der theoretischen Ausbeute.

Durch die Möglichkeit, ein kontinuierliches Verfahren durchführen zu können und der erzielbaren signifikant höheren Ausbeute, wird gegenüber den bisher, aufgrund des Feststoffanfalls erforderlichen diskontinuierlichen Verfahren eine deutliche Zunahme der Raum/Zeit-Ausbeute erzielt. Nicht-umgesetztes Amin kann dem Reaktionsapparat wieder rückgeführt werden, so dass die erzielbare Ausbeute beim erfindungsgemäßen Verfahren wesentlich höher liegt als bei den bekannten Verfahren.

## Patentansprüche

1. Verfahren zur Herstellung von Isocyanaten durch Umsetzung der entsprechenden primären Amine mit Phosgen in einem inerten Lösungsmittel bei einer Temperatur von 20 bis 200°C und einem Druck von 0,05 bis 0,5 MPa abs, **dadurch gekennzeichnet, dass** man 0,01 bis 50 Mol-% eines Sulfonsäureamids, bezogen auf die Gesamtmenge an primärem Amin und gebildetem Isocyanat in der Reaktionslösung, einsetzt.

2. Verfahren nach Anspruch 1, **dadurch gekennzeichnet, dass** man 0,01 bis 25 Mol-% des Sulfonsäureamids, bezogen auf die Gesamtmenge an primärem Amin und gebildetem Isocyanat in der Reaktionslösung, einsetzt.

3. Verfahren nach den Ansprüchen 1 bis 2, **dadurch gekennzeichnet, dass** man als Sulfonsäureamid ein Sulfonsäureamid der allgemeinen Formel (I)
R¹-SO₂-NH₂ (I),
in der der Rest R¹ einen unverzweigten oder verzweigten C₁-bis C₁₀-Alkylrest, einen unverzweigten oder verzweigten C₅-bis C₁₀-Cycloalkylrest oder einen unsubstituierten oder mit einem oder mehreren C₁- bis C₄-Alkylresten substituierten C₆-bis C₁₂-Aryl-Rest bedeutet, einsetzt.

4. Verfahren nach Anspruch 3, **dadurch gekennzeichnet, dass** man als Sulfonsäureamid (I) p-Toluolsulfonsäureamid einsetzt.

5. Verfahren nach den Ansprüchen 1 bis 4, **dadurch gekennzeichnet, dass** man als primäres Amin ein Monoamin mit einer NH₂-Gruppe oder ein Diamin mit zwei NH₂-Gruppen mit jeweils 1 bis 20 Kohlenstoffatomen einsetzt.

6. Verfahren nach Anspruch 5, **dadurch gekennzeichnet, dass** man als primäres Amin 1,6-Diaminohexan, Cyclohexylamin, Isophorondiamin, Anilin, Diaminotoluole, Diphenylmethan-4,4'-diamin, R-(+)- und S(-)-Phenylethylamin einsetzt.

7. Verfahren nach den Ansprüchen 1 bis 6, **dadurch gekennzeichnet, dass** man das Sulfonsäureamid in einem inerten Lösungmittel vorlegt, darin Phosgen einleitet und in die mit Phosgen beladene Ausgangslösung das primäre Amin zuführt.

8. Verfahren nach den Ansprüchen 1 bis 7, **dadurch gekennzeichnet, dass** man das inerte Lösungsmittel in einer Menge von 100 bis 2000 Gew.-%, bezogen auf die Gesamtmenge des vorliegenden primären Amins und des gebildeten Isocyanats, einsetzt.

9. Verfahren nach den Ansprüchen 1 bis 8, **dadurch gekennzeichnet, dass** man als inertes Lösungsmittel O-, m- oder p-Xylol, Chlorbenzol, O-, m-, p-Dichlorbenzol oder deren Gemische einsetzt.

## Claims

1. A process for preparing isocyanates by reacting the corresponding primary amines with phosgene in an inert solvent at from 20 to 200°C and a pressure of from 0.05 to 0.5 MPa abs, wherein from 0.01 to 50 mol% of a sulfonamide, based on the total amount of primary amine and isocyanate formed in the reaction solution, is used.

2. The process according to claim 1, wherein from 0.01 to 25 mol% of the sulfonamide, based on the total amount of primary amine and isocyanate formed in the reaction solution, is used.

3. The process according to claim 1 or 2, wherein a sulfonamide of the formula (I)
R¹ -SO₂-NH₂ (I),
is used as sulfonamide, where the radical R¹ is an unbranched or branched C₁-C₁₀-alkyl radical, an unbranched or branched C₅-C₁₀-cycloalkyl radical or a C₆-C₁₂-aryl radical which is unsubstituted or substituted by one or more C₁-C₄-alkyl radicals.

4. The process according to claim 3, wherein the sulfonamide (I) used is p-toluenesulfonamide.

5. The process according to any of claims 1 to 4, wherein the primary amine used is a monoamine having one NH₂ group or a diamine having two NH₂ groups, each having from 1 to 20 carbon atoms.

6. The process according to claim 5, wherein the primary amine used is 1,6-diaminohexane, cyclohexylamine, isophoronediamine, aniline, a diaminotoluene, diphenylmethane-4,4'-diamine, R-(+)- or S(-)-phenylethylamine.

7. The process according to any of claims 1 to 6, wherein the sulfonamide is initially charged in an inert solvent, phosgene is introduced therein and the primary amine is added to the phosgene-laden starting solution.

8. The process according to any of claims 1 to 7, wherein the inert solvent is used in an amount of from 100 to 2000% by weight, based on the total amount of primary amine present and isocyanate formed.

9. The process according to any of claims 1 to 8, wherein the inert solvent used is o-, m- or p-xylene, chlorobenzene, o-, m-, p-dichlorobenzene or a mixture thereof.

## Revendications

1. Procédé de fabrication d'isocyanates par le biais de la réaction des amines primaires correspondantes avec du phosgène dans un solvant inerte à une température de 20 à 200 °C et à une pression absolue de 0,05 à 0,5 MPa, **caractérisé en ce que** l'on utilise 0,01 à 50 % en mole d'un amide d'acide sulfonique par rapport à la quantité totale d'amine primaire et d'isocyanate formé dans la solution réactionnelle.

2. Procédé selon la revendication 1, **caractérisé en ce que** l'on utilise 0,01 à 25 % en mole de l'amide d'acide sulfonique par rapport à la quantité totale d'amine primaire et d'isocyanate formé dans la solution réactionnelle.

3. Procédé selon les revendications 1 à 2, **caractérisé en ce que** l'on utilise, comme amide d'acide sulfonique, un amide d'acide sulfonique de formule générale (I) :
R¹-SO₂-NH₂ (I),
dans laquelle le radical R¹ représente un radical alkyle en C₁-C₁₀ ramifié ou non ramifié, un radical cycloalkyle en C₅-C₁₀ ramifié ou non ramifié ou un radical aryle en C₆-C₁₂ non substitué ou substitué par un ou plusieurs radicaux alkyle en C₁-C₄.

4. Procédé selon la revendication 3, **caractérisé en ce que** l'on utilise, comme amide d'acide sulfonique (I), l'amide d'acide p-toluène-sulfonique.

5. Procédé selon les revendications 1 à 4, **caractérisé en ce que** l'on utilise, comme amine primaire, une monoamine avec un groupement NH₂ ou une diamine avec deux groupements NH₂ contenant, respectivement, 1 à 20 atomes de carbone.

6. Procédé selon la revendication 5, **caractérisé en ce que** l'on utilise, comme amine primaire, le 1,6-diaminohexane, la cyclohexylamine, l'isophoronediamine, l'aniline, des diaminotoluènes, la diphénylméthane-4,4'-diamine, la R-(+)- et la S(-)-phényléthylamine.

7. Procédé selon les revendications 1 à 6, **caractérisé en ce que** l'on incorpore l'amide d'acide sulfonique à un solvant inerte, **en ce que** l'on y introduit ensuite du phosgène et **en ce que** l'on achemine l'amine primaire à la solution de départ chargée en phosgène.

8. Procédé selon les revendications 1 à 7, **caractérisé en ce que** l'on utilise le solvant inerte en une quantité de 100 à 2000 % en poids par rapport à la quantité totale d'amine primaire présente et d'isocyanate formé.

9. Procédé selon les revendications 1 à 8, **caractérisé en ce que** l'on utilise, comme solvant inerte, le o-, le m- ou le p-xylène, le chloro-benzène, le o-, le m-, le p-dichlorobenzène ou leurs mélanges.
